# EUROPEAN PATENT APPLICATION

(11) **EP 3 467 506 A1**
(43) Date of publication of application: **10.04.2019**
(21) Application number: 17802830.4
(22) Date of filing: 24.05.2017
(51) Int. Cl.: G01N 33/68

(54) **METHOD FOR EVALUATING AND METHOD FOR MONITORING THERAPEUTIC EFFECT IN CRITICAL LIMB ISCHEMIA PATIENT AFTER ANGIOGENIC THERAPY, REAGENT FOR EVALUATING THERAPEUTIC EFFECT, AND DEVICE AND COMPUTER PROGRAM FOR EVALUATING THERAPEUTIC EFFECT**

(30) Priority: 26.05.2016 JP 2016105047
(71) Applicant: National University Corporation Nagoya University, Nagoya-shi, Aichi 464-8601 (JP)
(72) Inventor: KIKUCHI, Ryosuke, Nagoya-shi Aichi 464-8601 (JP); KONDO, Kazuhisa, Nagoya-shi Aichi 464-8601 (JP); MUROHARA, Toyoaki, Nagoya-shi Aichi 464-8601 (JP)
(74) Representative: TBK
(86) International application number: PCT/JP2017/019338
(87) International publication number: WO 2017/204244

(57) **Abstract**

Provided is a method whereby therapeutic effect in a critical limb ischemia patient after angiogenic therapy can be objectively evaluated with minimal patient burden.

The problem addressed by the present invention is solved by a method for evaluating a therapeutic effect in a critical limb ischemia patient who has undergone angiogenic therapy. The evaluation method includes, a measurement step for measuring a VEGF-A165b concentration in a blood of the critical limb ischemia patient and an evaluation step for evaluating the therapeutic effect taking changes over time in the measured VEGF-A165b concentration as an indicator.

## Description

### 1. Field of the Invention

The present invention relates to a method for evaluating and a method for monitoring the therapeutic effect in a critical limb ischemia patient who has undergone angiogenic therapy, a reagent for evaluating therapeutic effect, and a device and computer program for evaluating therapeutic effect, in particular to an evaluation method and monitoring method, a reagent for evaluating therapeutic effect, and a device and computer program for evaluating therapeutic effect that measure the concentration of VEGF-A₁₆₅b, an isoform of vascular endothelial growth factor-A (VEGF-A) expressed in the blood of critical limb ischemia patients, and take changes over time in the VEGF-A₁₆₅b concentration measured as an indicator.

### 2. Description of the Related Art

"Ischemic limb" is a disease in which the arteries in the arms or legs become clogged and narrow, causing blood circulation to deteriorate. Oral medication or injections and the like are commonly used in relatively mild cases such as coldness of the leg and calf pain when walking. On the other hand, limb ischemia is referred to as critical limb ischemia (CLI) when the symptoms progress to the point that the hands or feet hurt even at rest and develop sores (ulcers) at the ends. There are even cases in which the limb must be amputated if treatment is not performed to increase the blood circulation as much as possible.

Examples of major factors in the development of critical limb ischemia include peripheral artery disease (PAD) or arteriosclerosis obliterans (ASO), Buerger's disease or thromboangiitis obliterans (TAO), collagen disease-related angiitis (such as scleroderma and systemic lupus erythematosus), and the like.

PAD is a disease in which arteriosclerosis occurs and narrowing or clogging of an artery (peripheral artery) of a hand or foot causes blood flow to deteriorate, triggering various symptoms. The number of patients with PAD based on arteriosclerosis has tended to increase in Japan in recent years with westernization of the diet and aging of the population. Although the exact number of PAD patients is not currently known, some six million people are estimated to be affected based on epidemiological surveys and demographics in various parts of the country. Symptoms of PAD can include numbness and pain, skin ulcers as the condition worsens, and even necrosis of part of a limb. In addition, amputation of the affected limb (hand or foot) is necessary when the symptoms are severe, and the prognosis is poor.

Physical therapy, drug therapy, and revascularization are known as methods of treating critical limb ischemia. In particular, from the 1990s, attempts have been made to promote angiogenesis and collateral circulation from healthy tissues around the ischemic region using various growth factors, VEGF-A (vascular endothelial growth factor), and other such genes and human recombinant proteins. These treatments to promote angiogenesis and collateral circulation are called "therapeutic angiogenesis" or "angiogenic therapy."

As specific examples of angiogenic therapy, angiogenic therapy that transplants bone marrow mononuclear cells collected from bone marrow is known for critical limb ischemia in which limb amputation is unavoidable by conventional therapies (see Non-patent Document 1). Transplantation of adipose-derived regenerative cells (ADRCs) to the affected site is also known to enhance angiogenesis in the ischemic tissue (see Non-patent Document 2).

The following seven points are known as indicators for evaluating the therapeutic effect in critical limb ischemia (PAD, etc.) patients who have undergone angiogenic therapy.
1. Visual inspection: Photographically record ischemic ulcers that can be visually inspected.
2. Numerical rating scale (NRS): Evaluation of pain on 11 tiers of a scale from 0 to 10.
3. Measure the ankle-brachial index (ABI).
4. Measure the subcutaneous blood flow or skin tissue perfusion pressure (SPP) or percutaneous tissue oxygen partial pressure (TcO₂) by laser doppler.
5. Evaluate angiogenesis by diagnostic imaging.
6. Measure the CD34-positive cell count in the peripheral blood.
7. Measure the six-minute walking distance (additional item used only when a lower leg lesion permits walking).

### [Prior Art Documents]

### [Non-patent Documents]

[Non-patent Document 1] Eriko Tateishi-Yuyama et al., "Therapeutic Angiogenesis for Patients with Limb Ischemia by Autologous Transplantation of Bone Marrow Cells: Randomized Controlled Trial", The LANCET, Vol.360, August10, 2002, p427-435
[Non-patent Document 2] Kazuhisa Kando et al., "Implantation of Adipose-Derived Regenerative Cells Enhances Ischemia-Induced Angiogenesis.", Arterioscler Thromb Vasc Vasc Biol, January 2009, p61-66

### SUMMARY OF THE INVENTION

### PROBLEMS TO BE SOLVED BY THE INVENTION

Angiogenic therapy is expected to find further clinical application in the future as a method for treating critical limb ischemia patients. Nonetheless, only methods 1-7 listed above are known as indicators for evaluating the therapeutic effect in critical limb ischemia patients after they have undergone conventional angiogenic therapy. Pain and other such symptoms involve the subjectivity of the critical ischemic limb patient. Also, evaluation using equipment such as a laser doppler or diagnostic imaging requires the critical limb ischemia patient to go to where the equipment is located, and the patient burden is a significant problem. Even though the method of measuring the CD34-positive cell count in the peripheral blood does not involve the subjectivity of the critical limb ischemia patient and imposes little patient burden, the problem is that it mainly reflects how many of the transplanted cells have appeared in the blood and cannot evaluate the actual therapeutic effect. The development of a method that imposes little patient burden and permits objective evaluation of the therapeutic effect is therefore desired.

The present invention is an invention intended to solve the above problem of the prior art. It was newly discovered upon in-depth investigation that the therapeutic effect of angiogenic therapy can be evaluated by measuring the VEGF-A₁₆₅b concentration in the blood of a critical limb ischemia patient who has undergone angiogenic therapy and using the changes over time in the VEGF-A₁₆₅b concentration measured as an indicator, and the present invention was perfected.

Specifically, the purpose of the present invention is to provide an evaluation method and a monitoring method that permit evaluating the therapeutic effect in a critical limb ischemia patient who has undergone angiogenic therapy objectively and with little patient burden, a reagent for evaluating the therapeutic effect, and a device and computer program for evaluating the therapeutic effect.

### MEANS FOR SOLVING THE ABOVEMENTIONED PROBLEMS

The present invention, shown below, relates to a method for evaluating and method for monitoring the therapeutic effect in a critical limb ischemia patient who has undergone angiogenic therapy, a reagent for evaluating the therapeutic effect, and a device and computer program for evaluating the therapeutic effect.

(1) A method for evaluating a therapeutic effect in a critical limb ischemia patient who has undergone angiogenic therapy wherein the evaluation method includes:
   a measurement step for measuring a VEGF-A₁₆₅b concentration in a blood of the critical limb ischemia patient, and
   an evaluation step for evaluating the therapeutic effect taking changes over time in the measured VEGF-A₁₆₅b concentration as an indicator.
(2) The evaluation method according to (1) above, wherein:
   the evaluation step evaluates the therapeutic effect to be present when the VEGF-A₁₆₅b concentration rises temporarily after angiogenic therapy above that before angiogenic therapy, then decreases below that before angiogenic therapy.
(3) The evaluation method according to (1) above, wherein:
   the measurement step measures the VEGF-A₁₆₅b concentration and the total VEGF-A concentration in the blood of the critical limb ischemia patient, and
   the evaluation step uses the changes over time in the VEGF-A₁₆₅b concentration/total VEGF-A concentration ratio measured as the indicator.
(4) The evaluation method according to (1) or (2) above, wherein:
   the greater the percentage decrease in the VEGF-A₁₆₅b concentration a predetermined time after angiogenic therapy in comparison to the VEGF-A₁₆₅b concentration before angiogenic therapy, the higher the evaluation step evaluates the therapeutic effect to be.
(5) The evaluation method according to (3) above, wherein:
   the greater the percentage decrease in the VEGF-A₁₆₅b concentration/total VEGF-A concentration ratio a predetermined time after angiogenic therapy in comparison to the VEGF-A₁₆₅b concentration/total VEGF-A concentration before angiogenic therapy, the higher the evaluation step evaluates the therapeutic effect to be.
(6) The evaluation method according to (4) or (5) above, wherein:
   the evaluation step evaluates a therapeutic effect to be present when the percentage decrease is greater than a predetermined standard value.
(7) A method for monitoring a therapeutic effect in a critical limb ischemia patient who has undergone angiogenic therapy wherein the monitoring method includes:
   a first measurement step to measure a VEGF-A₁₆₅b concentration in a blood of the patient before angiogenic therapy,
   a second measurement step to measure the VEGF-A₁₆₅b concentration in the blood of the patient after angiogenic therapy, and
   a step to evaluate the therapeutic effect of angiogenic therapy in the patient based on the results of the first measurement step and the second measurement step.
(8) The monitoring method according to (7) above, wherein:
   a therapeutic effect of angiogenic therapy is evaluated to be present in the patient when the VEGF-A₁₆₅b concentration measured in the second measurement step is lower than the VEGF-A₁₆₅b concentration measured in the first measurement step.
(9) The monitoring method according to (7) above, wherein:
   VEGF-A₁₆₅a is also measured in the first measurement step,
   VEGF-A₁₆₅a is also measured in the second measurement step,
   a first ratio = (VEGF-A₁₆₅b concentration measured in first measurement step)/(total of VEGF-A₁₆₅a concentration and VEGF-A₁₆₅b concentration measured in first measurement step) is calculated prior to the evaluation step,
   a second ratio = (VEGF-A₁₆₅b concentration measured in second measurement step)/(total of VEGF-A₁₆₅a concentration and VEGF-A₁₆₅b concentration measured in second measurement step) is calculated prior to the evaluation step, and
   a therapeutic effect of angiogenic therapy is evaluated to be present in the patient when a value of the second ratio is lower than the value of the first ratio in the evaluation step.
(10) The monitoring method according to (7) above, wherein:
   total VEGF-A concentration is also measured in the first measurement step,
   total VEGF-A concentration is also measured in the second measurement step,
   a first ratio = (VEGF-A₁₆₅b concentration measured in first measurement step)/(total VEGF-A concentration measured in first measurement step) is calculated prior to the evaluation step,
   a second ratio = (VEGF-A₁₆₅b concentration measured in second measurement step)/(total VEGF-A concentration measured in second measurement step) is calculated prior to the evaluation step, and
   a therapeutic effect of angiogenic therapy is evaluated to be present in the patient when the value of the second ratio is lower than the value of the first ratio.
(11) The monitoring method according to any of (7)-(10) above, wherein:
   the second measurement step is performed approximately one month after angiogenic therapy.
(12) A reagent for evaluating a therapeutic effect in a critical limb ischemia patient that includes:
   a denaturing agent that breaks down the three-dimensional structure of at least the exon 8b portion of VEGF-A₁₆₅b, and
   an anti-VEGF-A₁₆₅b antibody.
(13) The reagent for evaluating the therapeutic effect in the critical limb ischemia patient according to (12) above, wherein:
   the denaturing agent is at least one selected from dithiothreitol, urea, and acid and alkali.
(14) A device for evaluating a therapeutic effect in a critical limb ischemia patient who has undergone angiogenic therapy equipped with a computer including a processor and memory under control of the processor in which
   the memory stores a computer program for the computer to execute:
   (1) a step that acquires a VEGF-A₁₆₅b concentration measured value and/or a VEGF-A₁₆₅b concentration/total VEGF-A concentration ratio in a blood of a critical limb ischemia patient,
   (2) a step that calculates a percentage decrease in the VEGF-A₁₆₅b concentration a predetermined time after angiogenic therapy to the VEGF-A₁₆₅b concentration prior to angiogenic therapy and/or the percentage decrease in the VEGF-A₁₆₅b concentration/total VEGF-A concentration ratio a predetermined time after angiogenic therapy to the VEGF-A₁₆₅b concentration/total VEGF-A concentration ratio prior to angiogenic therapy and compares the percentage decrease(s) calculated with predetermined standard value(s), and
   (3) a step that evaluates a therapeutic effect to be present in the critical limb ischemia patient who has undergone angiogenic therapy when the percentage decrease is greater than the predetermined standard value.
(15) A computer program that executes the following in a computer that includes a processor and memory under control of the processor:
   (1) a step that acquires a VEGF-A₁₆₅b concentration measured value and/or a VEGF-A₁₆₅b concentration/total VEGF-A concentration ratio in a blood of a critical limb ischemia patient,
   (2) a step that calculates the percentage decrease in the VEGF-A₁₆₅b concentration a predetermined time after angiogenic therapy to the VEGF-A₁₆₅b concentration prior to angiogenic therapy and/or the percentage decrease in the VEGF-A₁₆₅b concentration/total VEGF-A concentration ratio a predetermined time after angiogenic therapy to the VEGF-A₁₆₅b concentration/total VEGF-A concentration ratio prior to angiogenic therapy and compares the percentage decrease(s) calculated with predetermined standard value(s), and
   (3) a step that evaluates a therapeutic effect to be present in the critical limb ischemia patient who has undergone angiogenic therapy when the percentage decrease is greater than the predetermined standard value.

### ADVANTAGES OF THE INVENTION

The therapeutic effect of angiogenic therapy can be evaluated objectively with little patient burden by taking as an indicator the changes over time in the VEGF-A₁₆₅b concentration expressed in the blood of a critical limb ischemia patient after undergoing angiogenic therapy.

### BRIEF DESCRIPTION OF THE DRAWINGS

FIG. 1 shows the results of Western blotting analysis described in Non-patent Document 4.
FIG. 2 is a drawing outlining the structures of VEGF-A₁₆₅a and VEGF-A₁₆₅b.
FIG. 3 is a schematic drawing showing an example of a device for evaluating the therapeutic effect in a critical limb ischemia patient.
FIG. 4 is a block diagram showing a hardware configuration of an evaluation device.
FIG. 5 is a flow chart of an example of an embodiment of an evaluation method using an evaluation device.
FIG. 6 is photographs substituted for a drawing and is photographs taken before and after angiogenic therapy in the critical limb ischemia patients of cases 1-5.
FIG. 7(A) is a graph showing the changes over time in the VEGF-A₁₆₅b concentration in the blood of healthy subjects. FIG. 7(B) is a graph showing the changes over time in the VEGF-A₁₆₅b concentration in the blood of the critical limb ischemia patients of cases 1-5 before and after angiogenic therapy.
FIG. 8(A) is a graph showing the changes over time in the VEGF-A₁₆₅b concentration/total VEGF-A concentration ratio in the blood of healthy subjects. FIG. 8(B) is a graph showing the changes over time in the VEGF-A₁₆₅b concentration/total VEGF-A concentration ratio in the blood of the critical limb ischemia patients of cases 1-5 before and after angiogenic therapy.
FIG. 9(A) is a graph showing the changes over time in the total VEGF-A concentration in the blood of healthy subjects. FIG. 9(B) is a graph showing the changes over time in the total VEGF-A concentration in the blood of the critical limb ischemia patients of cases 1-5 before and after angiogenic therapy.
FIG. 10 is a graph showing the changes over time in the CD34-positive cell count in the blood of the critical limb ischemia patients of cases 1-5 before and after angiogenic therapy.
FIG. 11 is photographs substituted for a drawing and is a photograph of the critical limb ischemia patient of case 6 taken before angiogenic therapy and a photograph taken 28 days after therapy.
FIG. 12 is graphs showing the changes over time before and 28 days after angiogenic therapy of critical limb ischemia patients. FIG. 12(A) shows the changes over time in the total VEGF-A concentration; FIG. 12(B) shows the changes over time in the VEGF-A₁₆₅b concentration; FIG. 12(C) shows the changes over time in the VEGF-A₁₆₅b concentration/total VEGF-A concentration ratio.

### DESCRIPTION OF THE PREFERRED EMBODIMENTS

Embodiments of the method for evaluating the therapeutic effect in critical limb ischemia patients who have undergone angiogenic therapy (sometimes simply referred to hereinafter as the "evaluation method"), monitoring method, reagent for evaluating the therapeutic effect, and device and computer program for evaluating the therapeutic effect are explained in detail below.

The evaluation method shown in the embodiment takes as the indicator the changes over time in the VEGF-A₁₆₅b concentration expressed in the blood of a critical limb ischemia patient after undergoing angiogenic therapy (sometimes referred to hereinafter as "after surgery"). VEGF-A was identified in the 1980s and is known as a group of glycoproteins involved in blood vessel formation and angiogenesis. VEGF-A binds as a ligand to vascular endothelial growth factor receptors (VEGFR) on the surface of vascular endothelial cells where it acts to stimulate cell division, migration, and differentiation and to enhance the vascular permeability of the microvessels. It is also known to be involved in the activation of monocytes and macrophages. In addition to being involved in angiogenesis of the normal body, it is also known to be involved in the course of malignant transformation such as tumor blood vessel formation and metastasis.

On the other hand, VEGF-A₁₆₅b, which is in the VEGF-A family, was identified in 2002 as an isoform in which a portion of the sequences generated by specific splicing at the terminus of the 8th exon, which is a constituent element of the VEGF-A gene, are different (David O.B. et.al., "VEGF165b, an Inhibitory Splice Variant of Vascular Endothelial Growth Factor, Is Down-Regulated in Renal Cell Carcinoma.", Cancer Res. 2002 Jul 15; 62 (14) :4123-31) . VEGF-A₁₆₅b is known to have the effect of inhibiting VEGF-A-induced vascular endothelial proliferation, epithelial migration, vasodilation, in vivo angiogenesis, and tumor growth.

As regards the problem that ischemia of the lower limb tissue is not improving even though the blood concentration of VEGF-A which creates new blood vessels in PAD patients is rising dominantly in comparison to healthy persons (Clarence M. Findley et al., "Plasma Levels of Soluble Tie2 and Vascular Endothelial Growth Factor Distinguish Critical Limb Ischemia From Intermittent Claudication in Patients With Peripheral Arterial Disease", Journal of the American College of Cardiology, 2008, Vol.52, Issue5, p387-393, sometimes referred to hereinafter as "Non-patent Document 3"), the present inventors clarified by separately measuring the angiogenesis-promoting form VEGF-A₁₆₅a and the suppressing form VEGF-A₁₆₅b that VEGF-A₁₆₅b rises in PAD patients and that rise is a cause of angiogenesis insufficiency (R. Kikuchi et. Al., "An antiangiogenic isoform of VEGF-A contributes to impaired vascularization in peripheral artery disease" , Nature Medicine 20, P.1464-1471, (2014), sometimes referred to hereinafter as "Non-patent Document 4").

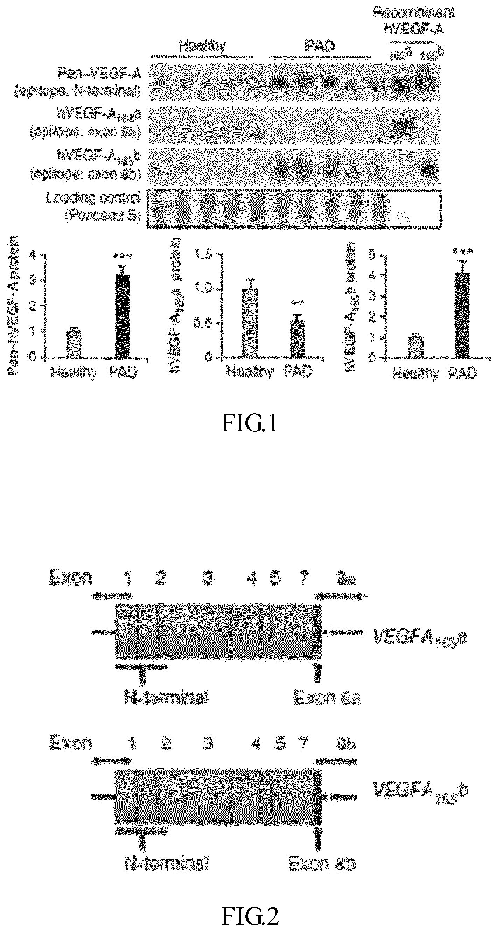

Furthermore, the above Non-patent Document 4 is a study conducted to elucidate the contradiction between the test results and the advance of the disease in PAD patients described in Non-patent Document 3. In addition, as shown in FIG.1, the experimental method described in Non-patent Document 4 only analyzed total VEGF-A, VEGF-A₁₆₅a, and VEGF-A₁₆₅b in the blood of healthy subjects and PAD patients by Western blotting and confirmed the magnitude of the expression levels as shown in FIG.1. As mentioned above, Non-patent Document 4 is a study focused on elucidating the contradiction between test results and advance of the disease in PAD patients and neither suggests nor describes accurately measuring the VEGF-A₁₆₅b concentration in the blood of critical limb ischemia patients after surgery and evaluating the therapeutic results in the critical limb ischemia patient after surgery by using the changes over time in the concentration measured as an indicator.

The term "angiogenic therapy" in the present specification means a therapeutic method for promoting the regeneration of blood vessels. Examples include, in addition to the transplantation of bone marrow mononuclear cells or adipose-derived regenerative cells to a patient mentioned above, methods that transplant vascular endothelial cells that produce VEGF-A, macrophages, smooth muscle, vascular endothelial progenitor cells, iPS cells, and the like to a patient.

FIG. 2 is a drawing outlining the structures of VEGF-A₁₆₅a and VEGF-A₁₆₅b (see Non-patent Document 4). The serum is separated from the blood by a conventional method, and the VEGF-A₁₆₅b concentration in the blood may be measured (measurement step) by enzyme immunoassay (ELISA) using a commercial anti-VEGF-A₁₆₅b antibody that specifically recognizes exon 8b. Furthermore, in the measurement step, a step that measures the VEGF-A₁₆₅b concentration in the blood of the patient before performing angiogenic therapy may be defined as a first measurement step and a step that measures the VEGF-A₁₆₅b concentration in the blood after performing angiogenic therapy may be defined as a second measurement step. When measuring the total VEGF-A concentration in the blood, the serum is separated from the blood by a conventional method and the total VEGF-A concentration may be measured by ELISA using a commercial anti-VEGF-A antibody that specifically recognizes the N-terminal shown in FIG. 2. When measuring the VEGF-A₁₆₅a concentration in the blood, the serum is separated from the blood by a conventional method, and the VEGF-A₁₆₅a concentration may be measured by ELISA using an anti-VEGF-A₁₆₅a antibody that specifically recognizes exon 8a. An anti-VEGF-A₁₆₅a antibody may be produced by a known method. The VEGF-A, VEGF-A₁₆₅a, and VEGF-A₁₆₅b concentrations in the blood can be measured accurately by ELISA.

Furthermore, VEGF-A₁₆₅b cannot be detected even if the VEGF-A₁₆₅b concentration in the blood (serum) is measured by ELISA using a commercial anti- VEGF-A₁₆₅b antibody. This is presumably because the three-dimensional structure of exon 8b to which the anti- VEGF-A₁₆₅b antibody binds differs depending on the sample (such as serum, plasma, urine, cell culture supernatant, etc.). Therefore, the present inventors newly discovered that the VEGF-A₁₆₅b concentration in the serum can be measured by ELISA using an anti-VEGF-A₁₆₅b antibody by conducting measurement after pretreating the serum separated from the blood. The pretreatment should be capable of breaking down the three-dimensional structure of at least the exon 8B portion of VEGF-A₁₆₅b. Examples include treating the serum by dithiothreitol (DTT) or urea or treating the serum by an acid such as hydrochloric acid and neutralizing by an alkali such as sodium hydroxide. Therefore, a reagent for evaluating the therapeutic effect in critical limb ischemia patients can be produced by combining (1) a denaturing agent that breaks down the three-dimensional structure of at least the exon 8b portion of VEGF-A₁₆₅b, selected from dithiothreitol, urea, and acid and alkali, etc., and (2) an anti-VEGF-A₁₆₅b antibody.

In the evaluation step, the VEGF-A₁₆₅b concentration in the blood (serum) is measured periodically at predetermined times, for example, after surgery, after 2 weeks, after 3 weeks, after 4 weeks, after 2 months, after 3 months, after 6 months, etc., even though the changes over time vary depending on the symptoms. A therapeutic effect can be evaluated to be present when the VEGF-A₁₆₅b concentration in the blood (serum) decreases significantly after a predetermined time in comparison to that before performing angiogenic therapy (sometimes referred to hereinafter as "before surgery"). Furthermore, as will be demonstrated in the examples described below, a temporary rise in the VEGF-A₁₆₅b concentration was seen in the blood of critical limb ischemia patients after surgery. Therefore, a therapeutic effect may be evaluated to be present when the VEGF-A₁₆₅b concentration rises temporarily after surgery and the VEGF-A₁₆₅b concentration subsequently decreases from that before surgery. Also, as will be demonstrated in the examples described below, the greater the percentage decrease in the VEGF-A₁₆₅b concentration, the greater the therapeutic effect can also be evaluated to be.

In the evaluation step, the changes over time in the VEGF-A₁₆₅b concentration in the blood (serum) may be taken as an indicator, but the VEGF-A₁₆₅b concentration and the total VEGF-A concentration in the blood of the critical limb ischemia patient may be measured after surgery and the changes over time in the VEGF-A₁₆₅b concentration/total VEGF-A concentration ratio measured may be taken as an indicator. Using the VEGF-A₁₆₅b concentration/total VEGF-A concentration ratio as an indicator allows one to understand the percentage of VEGF-A₁₆₅b in total VEGF-A and to understand whether the critical limb ischemia patient is in a state in which the blood vessels regenerate easily or a state that suppresses regeneration of the blood vessels. Furthermore, with regard to the above ratio, the VEGF-A₁₆₅b concentration/total VEGF-A concentration ratio in the blood of the patient before angiogenic therapy may be defined as the first ratio and the VEGF-A₁₆₅b concentration/total VEGF-A concentration ratio in the blood after angiogenic therapy may be defined as the second ratio. Also, as demonstrated in the examples described below, taking the ratio as an indicator allows one to predict the therapeutic effect accurately. The therapeutic effect in a critical limb ischemia patient after surgery may be evaluated taking the VEGF-A₁₆₅b concentration or the VEGF-A₁₆₅b concentration/total VEGF-A concentration ratio as an indicator, or by taking both the VEGF-A₁₆₅b concentration and the VEGF-A₁₆₅b concentration/total VEGF-A concentration ratio as indicators. Furthermore, methods for evaluating the therapeutic effect in a critical limb ischemia patient who has undergone angiogenic therapy are described above, but one can also use a monitoring method that observes the therapeutic effect in the patient while evaluating the therapeutic effect by using the above measurement step and evaluation step.

As described above, the therapeutic effect in a critical limb ischemia patient after surgery is evaluated using the VEGF-A₁₆₅b concentration or the VEGF-A₁₆₅b concentration/total VEGF-A concentration ratio as an indicator. However, VEGF-A is composed mainly of VEGF-A₁₆₅a and VEGF-A₁₆₅b. Therefore, the therapeutic effect in a critical limb ischemia patient after surgery can be evaluated even by using the VEGF-A₁₆₅a or the VEGF-A₁₆₅a concentration/total VEGF-A concentration ratio as an indicator. The VEGF-A₁₆₅a concentration may be measured by producing an antibody that specifically recognizes only VEGF-A₁₆₅a (e.g., that recognizes only exon 8a of FIG. 2) by a known method and measuring the VEGF-A₁₆₅a concentration by ELISA. Alternatively, the VEGF-A₁₆₅b concentration may be subtracted from the total VEGF-A concentration. Also, the VEGF-A₁₆₅b concentration/total of the VEGF-A₁₆₅b concentration and VEGF-A₁₆₅a concentration ratio may be used instead of the VEGF-A₁₆₅b concentration/total VEGF-A concentration ratio.

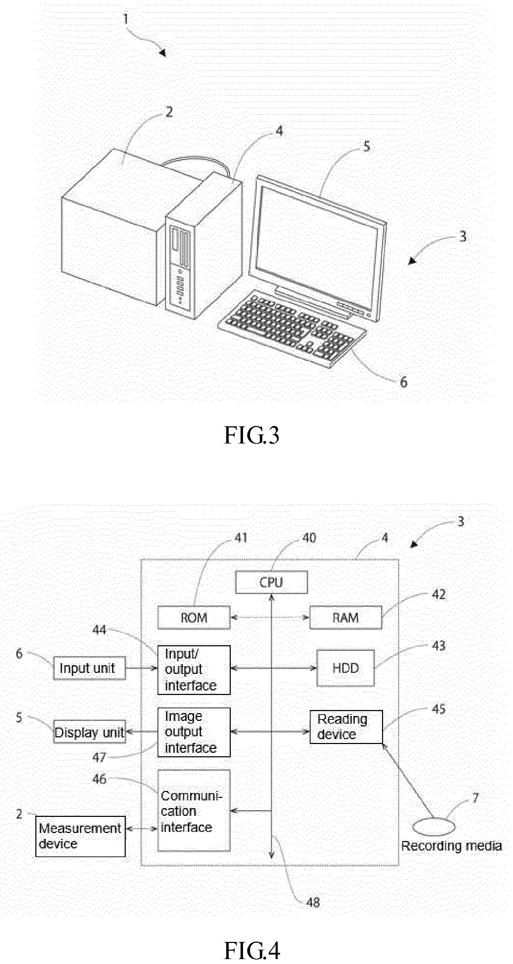

An embodiment of an evaluation device and evaluation program for implementing the evaluation method of the above embodiment is described below with reference to the drawings. FIG. 3 is a schematic diagram of an evaluation device 1. The evaluation device 1 includes a measurement device 2 and a computer system 3 connected to the measurement device 2.

The measurement device 2 measures the VEGF-A₁₆₅b concentration in the blood of a critical limb ischemia patient and, if necessary, the VEGF-A₁₆₅a concentration and total VEGF-A concentration. There are no particular limitations as to the measurement device 2, which can be selected as is appropriate in accordance with the method of measuring VEGF-A₁₆₅b, VEGF-A₁₆₅a, and VEGF-A. The measurement device 2 of this embodiment, for example, is a measurement device capable of detecting the signals produced by ELISA using an antibody that specifically recognizes only VEGF-A₁₆₅b, an antibody that specifically recognizes only VEGF-A₁₆₅a, and an antibody that specifically recognizes VEGF-A. Furthermore, labeled anti-VEGF-A₁₆₅b antibody, anti-VEGF-A₁₆₅a antibody, and anti-VEGF-A antibody may be used. Alternatively, a labeled secondary antibody that recognizes and binds with an anti-VEGF-A₁₆₅b antibody, anti-VEGF-A₁₆₅a antibody, or anti-VEGF-A antibody may also be used. Measurement devices of this type are not particularly restricted as long as they can detect signals based on the labels used and can be selected as is appropriate in accordance with the type of label.

When a reagent including at least the above antibody and a blood sample collected from a critical limb ischemia patient are set in the measurement device 2, the measurement device 2 carries out an antigen-antibody reaction using each reagent, acquires signals as optical information based on the antibodies bonded specifically to VEGF-A₁₆₅b, VEGF-A₁₆₅a, and VEGF-A, and sends the optical information obtained to a computer system 3. Furthermore, the blood sample may be a sample that has been denatured in advance by a denaturing agent that breaks down the three-dimensional structure of at least the exon 8b portion of VEGF-A₁₆₅b, or may be denatured using a denaturing agent inside the measurement device 2.

The computer system 3 may include a computer 4, an input unit 5 for inputting data, etc., and a display unit 6 for displaying information on the critical limb ischemia patient, evaluation results, etc. The computer system 3 acquires the VEGF-A₁₆₅b concentration measured value and/or the VEGF-A₁₆₅b concentration/total VEGF-A concentration ratio in a blood sample collected from a critical limb ischemia patient based on optical information received from measurement device 2. Next, (i) the percentage decrease in the VEGF-A₁₆₅b concentration (second measurement step) a predetermined time after angiogenic therapy to the VEGF-A₁₆₅b concentration (first measurement step) before angiogenic therapy and/or (ii) the percentage decrease in the ratio (second ratio) of the VEGF-A₁₆₅b concentration/total VEGF-A concentration ratio a predetermined time after angiogenic therapy to the ratio (first ratio) of the VEGF-A₁₆₅b concentration/total VEGF-A concentration ratio before angiogenic therapy is calculated. Next, the existence of a therapeutic effect in the critical limb ischemia patient who has undergone angiogenic therapy is evaluated by comparing the percentage decrease calculated to a predetermined standard value. Furthermore, the computer system 3, as shown in FIG. 3, may be a separate instrument from the measurement device 2 or may be incorporated into the measurement device 2.

The computer 4, as shown in FIG. 4, is equipped with a processor (CPU) 40, ROM 41, RAM 42, hard disk drive (HDD) 43, input/output interface 44, reading device 45, communication interface 46, and image output interface 47. These are connected so as to be able to communicate data by bus 48. Also, the measurement device 2 is connected so as to be able to communicate with computer 4 by communication interface 46.

The CPU 40 controls the operation of each input/output unit and executes computer programs stored in ROM 41 or on hard disk drive 43. In other words, the CPU 40 processes the optical information received from the measurement device 2 in accordance with a computer program, calculates the measured value of the VEGF-A₁₆₅b concentration in the blood sample and/or the VEGF-A₁₆₅b concentration/total VEGF-A concentration ratio, reads out the predetermined standard value (cutoff value) stored in ROM 41 or on hard disk drive 43, and evaluates that a therapeutic effect is present in the critical limb ischemia patient who has undergone angiogenic therapy when (i) the percentage decrease in the VEGF-A₁₆₅b concentration a predetermined time after angiogenic therapy to the VEGF-A₁₆₅b concentration before angiogenic therapy and/or (ii) the percentage decrease in the VEGF-A₁₆₅b concentration/total VEGF-A concentration ratio a predetermined time after angiogenic therapy to the VEGF-A₁₆₅b concentration/total VEGF-A concentration ratio before angiogenic therapy is greater than the standard value. The CPU 40 also outputs the determination results and displays them on the display unit 5.

ROM 41 is composed of a mask ROM, PROM, EPROM, EEPROM, etc. As described above, a computer program (evaluation program) for evaluating the therapeutic effect in a critical limb ischemia patient who has undergone angiogenic therapy executed by the CPU 40 and data used in executing this therapeutic effect evaluation program are recorded in ROM 41. Also, in addition to the predetermined standard value, the changes over time in the VEGF-A₁₆₅b concentration measured value of a critical limb ischemia patient and/or the VEGF-A₁₆₅b concentration/total VEGF-A concentration ratio, etc. may be recorded in ROM 41.

RAM 42 is composed of SRAM, DRAM, etc. RAM 42 is used in readout of the computer programs stored in ROM 41 and on the hard disk drive 43. RAM 42 is also utilized as a work area for the CPU 40 when the CPU 40 is executing these computer programs.

Computer programs for execution by the CPU 40 such as the operating system, application programs (therapeutic effect evaluation program), etc. and data used in the execution of these computer programs are stored on the hard disk drive 43. The hard disk drive 43 may record, in addition to the predetermined standard value (cutoff value), changes over time in the VEGF-A₁₆₅b concentration measured value of a critical limb ischemia patient and/or the VEGF-A₁₆₅b concentration/total VEGF-A concentration ratio, etc.

The reading device 45 is composed of a flexible disk drive, CD-ROM drive, DVD ROM drive, etc. The reading device 45 can read computer programs or data recorded by portable recording media 7.

The input/output interface 44 is composed, for example, of a USB, IEEE 1394, RS-232C, or other such serial interfaces and SCSI, IDE, IEEE 1284, or other such parallel interfaces, and an analog interface comprising a D/A converter, A/D converter, and the like. Input units 5 such as a keyboard, mouse, and the like are connected to the input/output interface 44. The operator can input various commands to the computer 3 by the input unit 5.

The communication interface 46 is, for example, an ethernet (registered trademark) interface or the like. The computer 3 can send printing data to a printer or the like by the communication interface 46.

The image output interface 47 is connected to the display unit 6 composed of an LCD, CRT, or the like. The display unit 6 can output a video signal corresponding to the image data given from the CPU 40. The display unit 6 displays an image according to the video signal input.

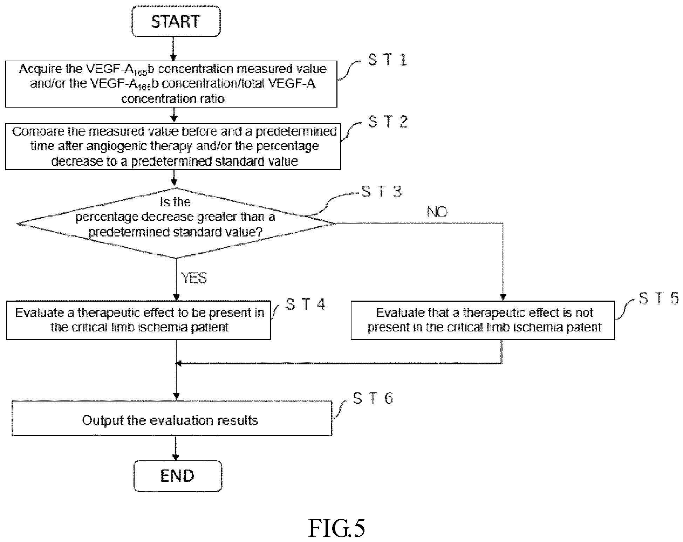

Next, an example of an embodiment of the method for evaluating the therapeutic effect in a critical limb ischemia patient who has undergone angiogenic therapy executed by the evaluation device 1 based on the evaluation program will be explained using FIG. 5. First, in step ST1, when optical information (signal) is received from the measurement device 2, the CPU 40 calculates the measured value of the VEGF-A₁₆₅b concentration and/or the VEGF-A₁₆₅b concentration/total VEGF-A concentration ratio in a blood sample based on the optical information acquired and stores it in ROM 41 or on the hard disk drive 43. In step ST2, the CPU 40 compares (i) the percentage decrease in the VEGF-A₁₆₅b concentration a predetermined time after angiogenic therapy to the VEGF-A₁₆₅b concentration before angiogenic therapy and/or (ii) the parentage decrease in the VEGF-A₁₆₅b concentration/total VEGF-A concentration ratio a predetermined time after angiogenic therapy to the VEGF-A₁₆₅b concentration/total VEGF-A concentration ratio before angiogenic therapy with a predetermined standard value (cutoff value) stored in ROM 41 or on the hard disk drive 43. When the percentage decrease is greater than the standard value (cutoff value), step ST3 becomes "YES" and the program advances to ST4. The CPU 40 evaluates that a therapeutic effect is present in the critical limb ischemia patient who has undergone angiogenic therapy and, in step ST6, the CPU 40 outputs the evaluation results, displays them on the display unit 5, and prints them by a printer. The evaluation result that there is a therapeutic effect may also be stored in ROM 41 or on the hard disk drive 43. On the other hand, when the percentage decrease is the same as or lower than the predetermined standard value (cutoff value), step ST3 becomes "NO" and the program advances to step ST5. The CPU 40 evaluates that a therapeutic effect is not present in the critical limb ischemia patent who has undergone angiogenic therapy. In step ST6, the CPU 40 outputs the evaluation results, displays them on the display unit 5, and prints them by a printer. The evaluation result that there is no therapeutic effect may also be stored in ROM 41 or on the hard disk drive 43. The VEGF-A₁₆₅b concentration/total VEGF-A concentration ratio may be substituted by the ratio of the VEGF-A₁₆₅b concentration/total value of the VEGF-A₁₆₅a concentration and VEGF-A₁₆₅b concentration in the therapeutic effect evaluation device and computer program.

An embodiment of the therapeutic effect evaluation device and computer program was explained above, but the present invention is not limited to the above embodiment and can be modified in various ways as long as one does not deviate from the spirit of the present invention.

The present invention is described more specifically below by using examples. The examples, however, are merely provided as a reference to specific embodiments to explain the present invention. These examples are to explain specific, concrete embodiments of the present invention, but in no way limit or restrict the scope of the invention disclosed herein.

### EXAMPLES

The clinical specimens, angiogenic therapy procedure, and method for measuring the VEGF-A₁₆₅b and total VEGF-A concentrations in the blood used in the examples and comparative examples are as follows.

### <Clinical specimens>

Angiogenic therapy was performed on critical limb ischemia patients of the following cases.
Case 1: 64-year-old female, scleroderma, intractable toe ulcer
Case 2: 65-year-old female, scleroderma + arteriosclerosis obliterans (ASO), intractable toe ulcer
Case 3: 49-year-old male, Buerger's disease, intractable to necrosis and ulcer
Case 4: 28-year-old female, scleroderma + systemic lupus erythematosus (SLE), intractable finger ulcer
Case 5: 37-year-old female, systemic lupus erythematosus (SLE), intractable toe ulcer

Five healthy subjects were also selected for comparison. The selection criteria for the healthy subjects were that they not satisfy the following four points.
1. Dyslipidemia
2. Impaired glucose tolerance
3. Smoking history
4. Abnormal blood pressure

### <Angiogenic therapy>

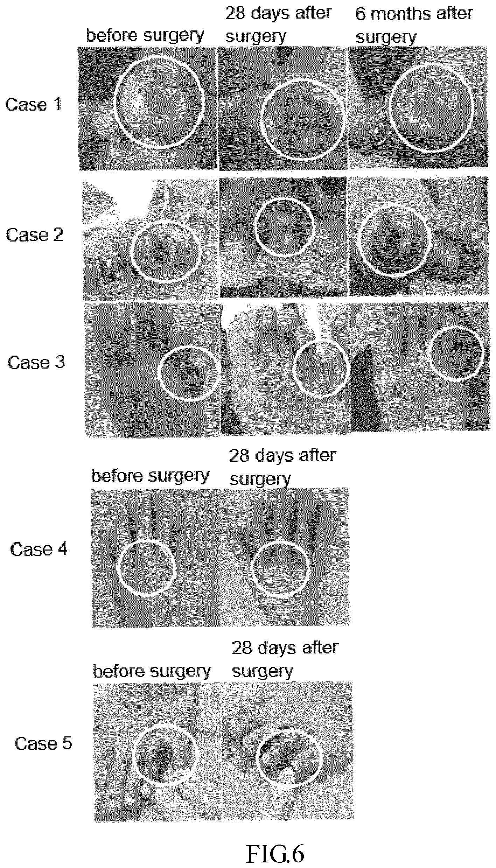

The critical limb ischemia patent was given local or general anesthesia, and approximately 300 mL of adipose tissue was collected from the patient's own subcutaneous fat by liposuction. Next, the liposuction suspension was placed in a special sterilized disposable kit connected to a Celution centrifugal separator (Cytori Therapeutics, Inc.), and ADRCs were isolated. The isolated ADRCs were transplanted to the affected site (ischemic limb) by intramuscular injection. FIG. 6 is photographs of the critical limb ischemia patients of cases 1-5 before and after (after 28 days and 6 months in cases 1-3, after 28 days in cases 4 and 5) surgery. As is evident from the photographs, angiogenic therapy improved the symptoms in all cases, although to different degrees.

### <Measurement of VEGF-A₁₆₅b concentration in blood>

Serum was first separated from the blood by a conventional method. Next, DTT was added to make a final concentration of 5 mM to the isolated serum, and pretreatment was carried out for 60 minutes at 37°C. Next, the VEGF-A₁₆₅b concentration in the pretreated blood was measured using a Human Vascular Endothelial Growth Factor-165b ELISA Kit (MyBioSource Inc., MBS720132) according to the manufacturer's instructions.

### <Measurement of total VEGF-A concentration in blood>

Serum was first separated from the blood by a conventional method. Next, the total VEGF-A concentration in the serum was measured using a Human VEGF Quantikine ELISA Kit, DVE00 (R&D Inc) according to the manufacturer's instructions.

### <Measurement of CD34-positive cell count in blood>

The CD34-positive cell count in the blood was measured using a Beckman Coulter A07776 according to the attached manual.

### <Example 1 and Comparative Example 1>

### [Changes over time in VEGF-A₁₆₅b concentration]

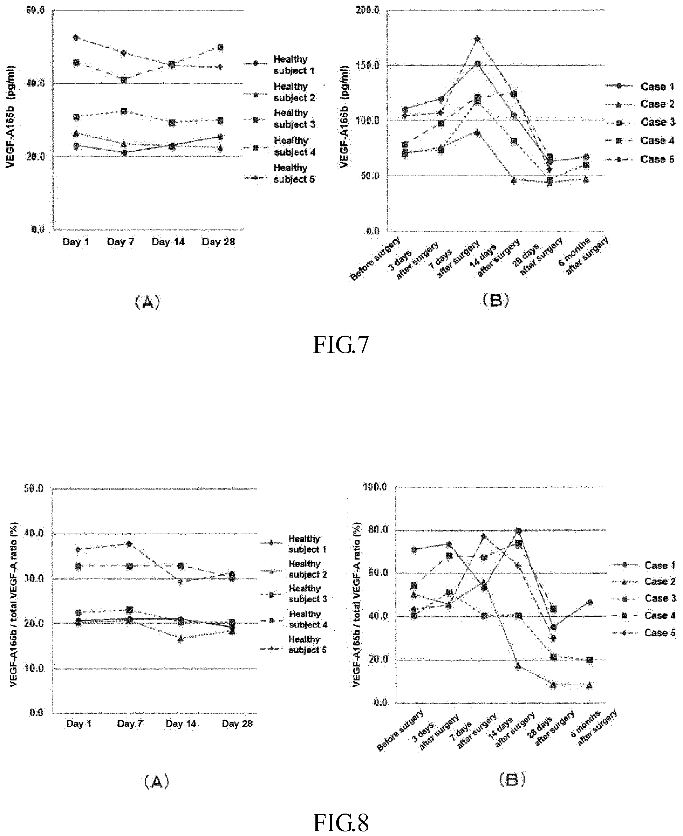

FIG. 7(A) is a graph showing the changes over time in the VEGF-A₁₆₅b concentration in the blood of healthy subjects, and FIG. 7(B) is a graph showing the changes over time in the VEGF-A₁₆₅b concentration in the blood before and after surgery in the critical limb ischemia patients of cases 1-5. As shown in FIG. 7(A), no significant trend could be seen in healthy subjects with a mean change in the VEGF-A₁₆₅b concentration of -2.9%±12.2% (p = 0.8125) from day 1 to day 28. On the other hand, as shown in FIG. 7(B), the VEGF-A₁₆₅b concentration in the blood on day 28 after surgery decreased in all cases in comparison to before surgery in the critical limb ischemia patients of cases 1-5, and a significant trend was seen with a mean concentration change -35.0±12.4% (p = 0.0114). Also, as shown by cases 1-3 in FIG. 7(B), the VEGF-A₁₆₅b concentration in the blood 6 months after surgery was the same or very slightly increased in comparison to 28 days after surgery, but still sufficiently lower than the VEGF-A₁₆₅b concentration before surgery. Furthermore, the rise in the VEGF-A₁₆₅b concentration in the blood on days 3 and 7 after surgery is thought to be because of inflammatory cells surrounding the transplantation site due to angiogenic therapy and VEGF-A₁₆₅b being released into the blood as a secretion of inflammatory cells. As is also evident from FIG. 7(B), the VEGF-A₁₆₅b concentration in the blood was decreased from that before surgery on day 14 after surgery in cases 1 and 2.

The above results clarified that the therapeutic effect of angiogenic therapy can be evaluated using the changes over time in the VEGF-A₁₆₅b concentration in the blood of a critical limb ischemia patient after surgery as an indicator. Also, despite differences depending on the case, the changes over time in the VEGF-A₁₆₅b concentration are monitored for at least 14 days after surgery, and a therapeutic effect can be evaluated to be present when the VEGF-A₁₆₅b concentration in the blood is decreased from before surgery.

In addition, there is little change in the VEGF-A₁₆₅b concentration in the blood even though differences are seen in the visual therapeutic effect 28 days and 6 months after surgery, as shown by the photographs of cases 1-3 in FIG. 6. Therefore, since using the evaluation method of the present invention permits a treatment prediction at a relatively early stage after surgery, the treatment motivation of the critical limb ischemia patient can be raised and treatment plans such as additional angiogenic therapy can be reexamined at an early stage.

### <Example 2 and Comparative Example 2>

### [Changes over time in the VEGF-A₁₆₅b concentration/total VEGF-A concentration ratio]

FIG. 8(A) is a graph showing the changes over time in the VEGF-A₁₆₅b concentration/total VEGF-A concentration ratio in the blood of healthy subjects. FIG. 8(B) is a graph showing the changes over time in the VEGF-A₁₆₅b concentration/total VEGF-A concentration ratio in the blood of the critical limb ischemia patients of cases 1-5 before and after surgery. As shown in FIG. 8(A), no significant trend was seen in the VEGF-A₁₆₅b concentration/total VEGF-A concentration ratio in the blood from day 1 to day 28 in healthy subjects with a mean change of -9.9%±2.9% (p = 0.063). On the other hand, as shown in FIG. 8(B), the VEGF-A₁₆₅b concentration/total VEGF-A concentration ratio in the blood on day 28 after surgery decreased in all cases in comparison to before surgery in the critical limb ischemia patients of cases 1-5, and a significant trend was seen with a mean change in the ratio of -46.2±23.7% (p = 0.0172).

Also, as shown by the photographs taken after 6 months in cases 1-3 in FIG. 6, an ulcerated part still remained even though a therapeutic effect was seen in case 1, but the ulcerated part virtually disappeared in cases 2 and 3, showing a high therapeutic effect. However, if we examine the changes over time in the VEGF-A₁₆₅b concentration 28 days and 6 months after surgery in cases 1-3 in FIG. 7(B), the VEGF-A₁₆₅b concentration is slightly higher 6 months after surgery in all cases. On the other hand, if we examine the changes over time in the VEGF-A₁₆₅b concentration/total VEGF-A concentration ratio 28 days and 6 months after surgery in cases 1-3 in FIG. 8(B), the ratio was higher 6 months after surgery in case 1, but the ratio was lower 6 months after surgery in cases 2 and 3, and the amount of the total VEGF-A concentration occupied by the VEGF-A₁₆₅b concentration was also lower in cases 2 and 3 than in case 1.

The above results suggested that the therapeutic effect can be evaluated (predicted) more accurately using the changes over time in the VEGF-A₁₆₅b concentration/total VEGF-A concentration ratio as an indicator.

### <Comparative Examples 3 and 4>

### [Changes over time in total VEGF-A concentration]

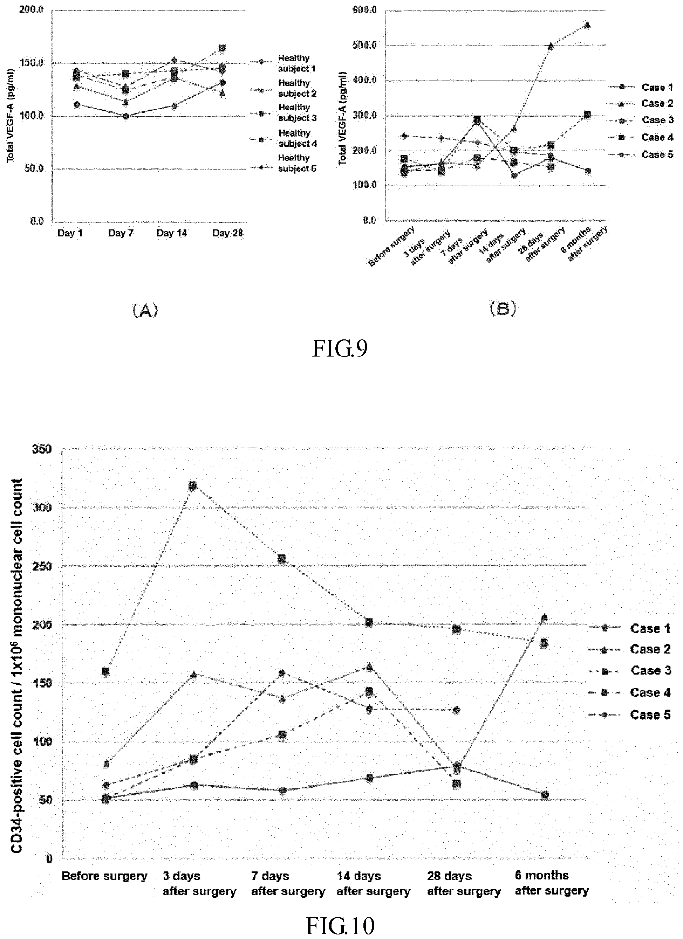

FIG. 9(A) is a graph showing the changes over time in the total VEGF-A concentration on the blood of healthy subjects. FIG. 9(B) is a graph showing the changes over time in the total VEGF-A concentration in the blood of the critical limb ischemia patents of cases 1-5 before and after surgery. No significant trend was seen in the total VEGF-A concentration in the blood from day 1 to day 28 in healthy subjects shown in FIG. 9(A) with a mean change of 7.6%±10.9% (p = 0.4127). Also, variations were found in the total VEGF-A concentration in the blood from day 1 to day 28 in the critical limb ischemia patients of cases 1-5 shown in FIG. 9(B) with a mean change of 56.8±115.6% (p = 3558), and no significant trend could be seen.

### <Comparative Example 5>

FIG. 10 is a graph showing the changes over time in the CD34-positive cell count in the critical limb ischemia patients of cases 1-5 before and after surgery. No significant trend was seen in the CD34-positive cell count from day 1 to day 28 in the critical limb ischemia patients of cases 1-5 with a mean change of 38.4±41.4% (p = 0.2857).

As described above, it became clear that the therapeutic effect of angiogenic therapy could be evaluated using the changes over time in the VEGF-A₁₆₅b concentration and the changes over time in the VEGF-A₁₆₅b concentration/total VEGF-A concentration ratio in specimens from cases 1 to 5 as an indicator. Furthermore, based on the photographs before and after treatment in FIG. 6 and on FIG. 7(B) and FIG. 8(B), the trend was seen that the greater the percentage decrease in the VEGF-A₁₆₅b concentration and the VEGF-A₁₆₅b concentration/total VEGF-A concentration ratio on day 28 after surgery in comparison to before surgery, the higher the therapeutic effect. This was therefore studied further.

### <Examples 3 and 4 and Comparative Example 6>

Angiogenic therapy was performed by the same procedure as above on a critical limb ischemia patient of the following case, and the changes over time in the VEGF-A₁₆₅b concentration (Example 3), changes over time in the VEGF-A₁₆₅b concentration/total VEGF-A concentration ratio (Example 4), and the changes over time in the total VEGF-A concentration (Comparative Example 6) were measured before and 28 days after surgery.

Case 6: 43-year-old male, Buerger's disease, intractable toe necrosis and ulcer

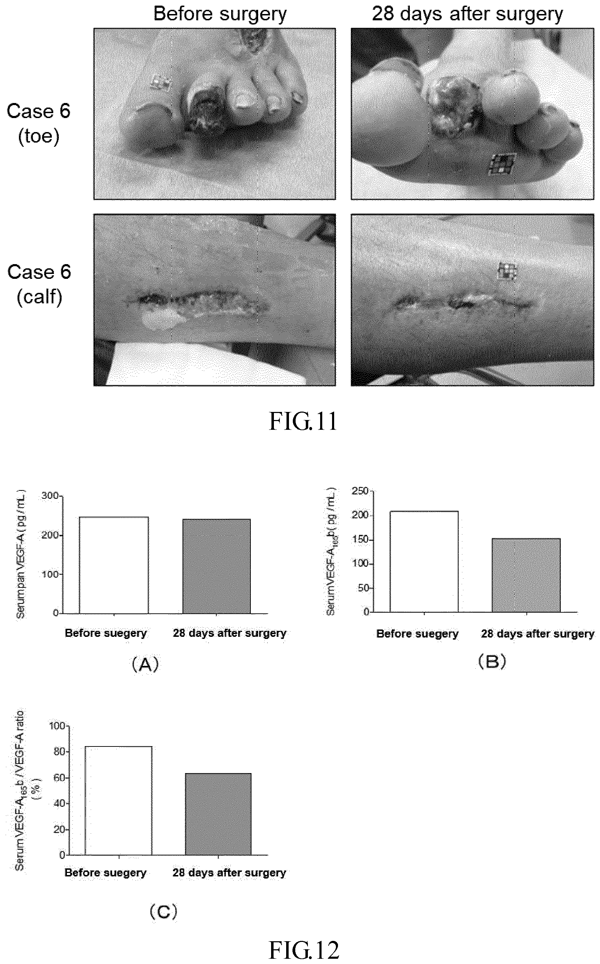

FIG. 11 is photographs of the critical limb ischemia patient of case 6 before and 28 days after surgery. Furthermore, case 6 had ulcers on the toe and calf. As is evident from the photographs, the therapeutic effect was slight even though the ulcer on the calf became smaller and the symptoms improved due to angiogenic therapy.

FIG. 12 is a graph showing the changes over time before and 28 days after surgery. FIG. 12(A) shows the changes over time in the total VEGF-A concentration; FIG. 12(B) shows the changes over time in the VEGF-A₁₆₅b concentration; and FIG. 12(C) shows the changes over time in the VEGF-A₁₆₅b concentration/total VEGF-A concentration ratio. Although virtually no changes over time were seen in the total VEGF-A concentration, the VEGF-A₁₆₅b concentration and the VEGF-A₁₆₅b concentration/total VEGF-A concentration ratio were decreased significantly 28 days after surgery in comparison to before surgery.

Next, the numerical values and percentage changes before and 28 days after surgery from each graph of FIG. 7(B), FIG. 8(B), FIG. 9(B), and FIG. 12(A)-(C) were summarized in tables. Also, the therapeutic effect was classified as marked improvement, moderate improvement, or slight improvement based on the photographs of FIG. 6 and FIG. 11.

**[Table 1]**

| <Total VEGF-A (pg/mL)> | | | | | |
|---|---|---|---|---|---|
| | Before surgery | 28 days after surgery | Percentage change | Affected limb | Therapeutic effect |
| Case 1 | 155.2 | 179.3 | 15.5 | right leg | moderate |
| Case 2 | 138.6 | 500.6 | 261.3 | right leg | marked |
| Case 3 | 177.8 | 218.0 | 22.6 | left leg | marked |
| Case 4 | 144.4 | 155.2 | 7.5 | right leg | marked |
| Case 5 | 242.4 | 187.2 | -22.8 | left leg | marked |
| Case 6 | 246.5 | 240.6 | -2.4 | left leg | slight |

**[Table 2]**

| <VEGF-A165b (pg/mL)> | | | | | |
|---|---|---|---|---|---|
| | Before surgery | 28 days after surgery | Percentage change | Affected limb | Therapeutic effect |
| Case 1 | 110.3 | 63.1 | -42.7 | right leg | moderate |
| Case 2 | 70.0 | 44.5 | -36.5 | right leg | marked |
| Case 3 | 72.4 | 46.9 | -35.3 | left leg | marked |
| Case 4 | 78.9 | 67.6 | -14.3 | right leg | marked |
| Case 5 | 104.8 | 56.2 | -46.4 | left leg | marked |
| Case 6 | 208.0 | 152.6 | -26.6 | left leg | slight |

**[Table 3]**

| <VEGF-A165b/Pan VEGF-A ratio (%)> | | | | | |
|---|---|---|---|---|---|
| | Before surgery | 28 days after surgery | Percentage change | Affected limb | Therapeutic effect |
| Case 1 | 71.0 | 35.2 | -50.4 | right leg | moderate |
| Case 2 | 50.5 | 8.9 | -82.4 | right leg | marked |
| Case 3 | 40.7 | 21.5 | -47.2 | left leg | marked |
| Case 4 | 54.6 | 43.6 | -20.3 | right leg | marked |
| Case 5 | 43.2 | 30.0 | -30.6 | left leg | marked |
| Case 6 | 84.4 | 63.4 | -24.8 | left leg | slight |

As is evident from Table 1, no correlation was seen between the percentage change in the changes over time in the total VEGF-A concentration and the therapeutic effect. On the other hand, as is evident from Tables 2 and 3, the percentage change (percentage decrease) in the changes over time in the VEGF-A₁₆₅b concentration and the percentage change (percentage decrease) in the changes over time in the VEGF-A₁₆₅b concentration/total VEGF-A concentration ratio of case 6 were smaller than in cases 1-3 and 5, and the therapeutic effect was also slight. Furthermore, case 4 had a marked therapeutic effect despite the small percentage changes (percentage decreases) shown in Table 2 and 3. This is thought to be because the affected site was an arm (hand) only in case 4, different from the affected site (leg) of the other cases.

Based on the above results, the VEGF-A₁₆₅b concentration and the VEGF-A₁₆₅b concentration/total VEGF-A concentration ratio decreased after a predetermined time (28 days after surgery) in comparison to before surgery in all cases. Therefore, for example, setting the standard value (cutoff value) of the percentage decrease at 0, a therapeutic effect can be evaluated to be present when the percentage decrease is greater than 0. Also, the trend obtained was that the greater the percentage decrease, the greater the therapeutic effect was. Therefore, a therapeutic effect is evaluated to be present when the percentage decrease after a predetermined time (for example, approximately one month after surgery or the like) is greater than 30%, and the need for retreatment can be evaluated clearly by setting the standard value in advance. It was also clarified that the standard value may be different for the arms and legs.

Based on the above results, it was clarified that the therapeutic effect can be evaluated in critical limb ischemia patients who have undergone angiogenic therapy using the changes over time in the VEGF-A₁₆₅b concentration in the blood of critical limb ischemia patients after surgery as an indicator.

### INDUSTRIAL APPLICABILITY

The present invention makes it possible to evaluate the therapeutic effect in critical limb ischemia patients who have undergone angiogenic therapy objectively with little patient burden. The present invention is therefore useful in the medical industry.

## Claims

1. A method for evaluating a therapeutic effect in a critical limb ischemia patient who has undergone angiogenic therapy wherein the evaluation method includes:
a measurement step for measuring a VEGF-A₁₆₅b concentration in a blood of the critical limb ischemia patient, and
an evaluation step for evaluating the therapeutic effect taking changes over time in the measured VEGF-A₁₆₅b concentration as an indicator.

2. The evaluation method according to claim 1, wherein:
the evaluation step evaluates the therapeutic effect to be present when the VEGF-A₁₆₅b concentration rises temporarily after angiogenic therapy above that before angiogenic therapy, then decreases below that before angiogenic therapy.

3. The evaluation method according to claim 1, wherein:
the measurement step measures the VEGF-A₁₆₅b concentration and the total VEGF-A concentration in the blood of the critical limb ischemia patient, and
the evaluation step uses the changes over time in the VEGF-A₁₆₅b concentration/total VEGF-A concentration ratio measured as the indicator.

4. The evaluation method according to claims 1 or 2, wherein:
the greater the percentage decrease in the VEGF-A₁₆₅b concentration a predetermined time after angiogenic therapy in comparison to the VEGF-A₁₆₅b concentration before angiogenic therapy, the higher the evaluation step evaluates the therapeutic effect to be.

5. The evaluation method according to claim 3, wherein:
the greater the percentage decrease in the VEGF-A₁₆₅b concentration/total VEGF-A concentration ratio a predetermined time after angiogenic therapy in comparison to the VEGF-A₁₆₅b concentration/total VEGF-A concentration before angiogenic therapy, the higher the evaluation step evaluates the therapeutic effect to be.

6. The evaluation method according to claims 4 or 5, wherein:
the evaluation step evaluates a therapeutic effect to be present when the percentage decrease is greater than a predetermined standard value.

7. A method for monitoring a therapeutic effect in a critical limb ischemia patient who has undergone angiogenic therapy wherein the monitoring method includes:
a first measurement step to measure a VEGF-A₁₆₅b concentration in a blood of the patient before angiogenic therapy,
a second measurement step to measure the VEGF-A₁₆₅b concentration in the blood of the patient after angiogenic therapy, and
a step to evaluate the therapeutic effect of angiogenic therapy in the patient based on the results of the first measurement step and the second measurement step.

8. The monitoring method according to claim 7, wherein:
a therapeutic effect of angiogenic therapy is evaluated to be present in the patient when the VEGF-A₁₆₅b concentration measured in the second measurement step is lower than the VEGF-A₁₆₅b concentration measured in the first measurement step.

9. The monitoring method according to claim 7, wherein: VEGF-A₁₆₅a is also measured in the first measurement step, VEGF-A₁₆₅a is also measured in the second measurement step,
a first ratio = (VEGF-A₁₆₅b concentration measured in first measurement step)/(total of VEGF-A₁₆₅a concentration and VEGF-A₁₆₅b concentration measured in first measurement step) is calculated prior to the evaluation step,
a second ratio = (VEGF-A₁₆₅b concentration measured in second measurement step)/(total of VEGF-A₁₆₅a concentration and VEGF-A₁₆₅b concentration measured in second measurement step) is calculated prior to the evaluation step, and
a therapeutic effect of angiogenic therapy is evaluated to be present in the patient when a value of the second ratio is lower than the value of the first ratio in the evaluation step.

10. The monitoring method according to claim 7, wherein:
total VEGF-A concentration is also measured in the first measurement step,
total VEGF-A concentration is also measured in the second measurement step,
a first ratio = (VEGF-A₁₆₅b concentration measured in first measurement step)/(total VEGF-A concentration measured in first measurement step) is calculated prior to the evaluation step,
a second ratio = (VEGF-A₁₆₅b concentration measured in second measurement step)/(total VEGF-A concentration measured in second measurement step) is calculated prior to the evaluation step, and
a therapeutic effect of angiogenic therapy is evaluated to be present in the patient when the value of the second ratio is lower than the value of the first ratio.

11. The monitoring method according to any of claims 7-10, wherein:
the second measurement step is performed approximately one month after angiogenic therapy.

12. A reagent for evaluating a therapeutic effect in a critical limb ischemia patient that includes:
a denaturing agent that breaks down the three-dimensional structure of at least the exon 8b portion of VEGF-A₁₆₅b, and
an anti-VEGF-A₁₆₅b antibody.

13. The reagent for evaluating the therapeutic effect in the critical limb ischemia patient according to claim 12, wherein:
the denaturing agent is at least one selected from dithiothreitol, urea, and acid and alkali.

14. A device for evaluating a therapeutic effect in a critical limb ischemia patient who has undergone angiogenic therapy equipped with a computer including a processor and memory under control of the processor in which
the memory stores a computer program for the computer to execute:
(1) a step that acquires a VEGF-A₁₆₅b concentration measured value and/or a VEGF-A₁₆₅b concentration/total VEGF-A concentration ratio in a blood of a critical limb ischemia patient,
(2) a step that calculates a percentage decrease in the VEGF-A₁₆₅b concentration a predetermined time after angiogenic therapy to the VEGF-A₁₆₅b concentration prior to angiogenic therapy and/or the percentage decrease in the VEGF-A₁₆₅b concentration/total VEGF-A concentration ratio a predetermined time after angiogenic therapy to the VEGF-A₁₆₅b concentration/total VEGF-A concentration ratio prior to angiogenic therapy and compares the percentage decrease(s) calculated with predetermined standard value(s), and
(3) a step that evaluates a therapeutic effect to be present in the critical limb ischemia patient who has undergone angiogenic therapy when the percentage decrease is greater than the predetermined standard value.

15. A computer program that executes the following in a computer that includes a processor and memory under control of the processor:
(1) a step that acquires a VEGF-A₁₆₅b concentration measured value and/or a VEGF-A₁₆₅b concentration/total VEGF-A concentration ratio in a blood of a critical limb ischemia patient,
(2) a step that calculates the percentage decrease in the VEGF-A₁₆₅b concentration a predetermined time after angiogenic therapy to the VEGF-A₁₆₅b concentration prior to angiogenic therapy and/or the percentage decrease in the VEGF-A₁₆₅b concentration/total VEGF-A concentration ratio a predetermined time after angiogenic therapy to the VEGF-A₁₆₅b concentration/total VEGF-A concentration ratio prior to angiogenic therapy and compares the percentage decrease(s) calculated with predetermined standard value(s), and
(3) a step that evaluates a therapeutic effect to be present in the critical limb ischemia patient who has undergone angiogenic therapy when the percentage decrease is greater than the predetermined standard value.
